# EUROPEAN PATENT SPECIFICATION

(11) **EP 4 221 648 B1**
(45) Date of publication and mention of the grant of the patent: **09.10.2024**
(21) Application number: 21794189.7
(22) Date of filing: 28.09.2021
(51) Int. Cl.: A61F 9/007

(54) **ERGONOMIC PHACOEMULSIFICATION HANDPIECE WITH ROTATING NEEDLE**
ERGONOMISCHES PHAKOEMULSIFIKATIONSHANDSTÜCK MIT ROTIERENDER NADEL
PIÈCE À MAIN ERGONOMIQUE DE PHACOÉMULSIFICATION AVEC AIGUILLE ROTATIVE

(30) Priority: 30.09.2020 US 202063085411 P
(43) Date of publication of application: 09.08.2023
(73) Proprietor: Johnson & Johnson Surgical Vision, Inc., Irvine, CA 92618 (US)
(72) Inventor: BRADY, John, Irvine, CA 92618 (US)
(74) Representative: Carpmaels & Ransford LLP
(86) International application number: PCT/IB2021/058839
(87) International publication number: WO 2022/070030

(56) References cited:
- WO-A2-2011/008672
- US-A1- 2005 277 970
- US-A1- 2008 294 087
- US-A1- 2009 005 712
- US-A1- 2010 069 825
- US-A1- 2016 038 342
- US-A1- 2019 321 017

## Description

### FIELD OF THE DISCLOSURE

The field of the invention relates to a handpiece, and more particularly to an apparatus, system and method for ergonomic phacoemulsification handpieces.

### BACKGROUND OF THE DISCLOSURE

A cataract is an opacity that develops in the lens of an eye. Cataracts are the most significant cause of blindness worldwide. Phacoemulsification is a medically recognized technique utilized for crystalline lens removal, which is a highly prevalent method of treating cataracts.

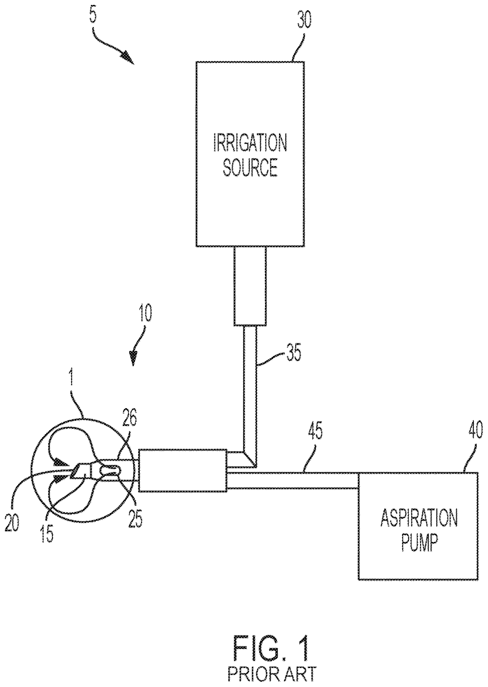

Phacoemulsification includes emulsifying, or liquefying, the cataractic lens through a corneal and/or scleral incision. A phacoemulsification system 5 known in the art is shown in FIG. 1. The system 5 generally includes a phacoemulsification handpiece 10 coupled to an irrigation source 30 and more or more aspiration pumps, e.g. pump 40, for insertion into the eye through the incision.

The handpiece 10 includes a distal tip (i.e., a needle) 15 (shown within the anterior chamber of the patient's eye 1) that emits ultrasonic energy to emulsify the cataractic lens within the patient's eye 1. The handpiece 10 further includes: a sleeve 26 that surrounds at least a portion of needle 15, and which provides one or more irrigation ports 25 proximal to the distal tip 15 that are coupled to an irrigation source 30 via an irrigation line 35; and an aspiration port 20 at the distal tip 15 which is coupled to aspiration pump 40 via an aspiration line 45. Concomitantly with the emulsification, fluid from the irrigation source 30, which is typically an elevated bottle of saline solution, is irrigated into the eye 1 via the irrigation line 35 and the irrigation port 25, and the irrigation fluid and emulsified cataractic lens material are aspirated from the eye 1 by the aspiration pump 40 via the aspiration port 20 and the aspiration line 45.

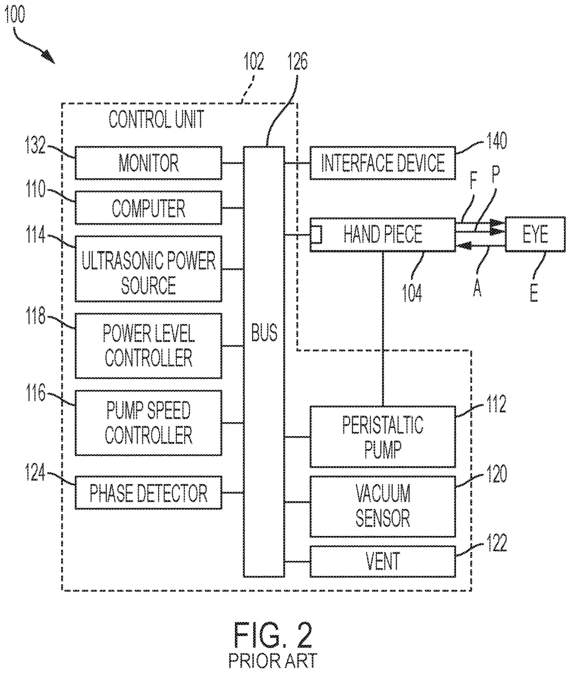

Turning to FIG. 2, a functional block diagram of a phacoemulsification system 100 known in the art is shown. The system 100 includes a control unit 102 and a handpiece 104 operably coupled together. The control unit 102 generally controls the operating parameters of the handpiece 104, e.g., the rate of aspiration A, rate of irrigation (or flow) F, and power P applied to the needle, and hence the eye E. The control unit 102 generally includes a microprocessor computer 110 which is operably connected to and controls the various other elements of the system 100.

The control unit 102 may include an aspiration pump, such as a Venturi (or vacuum-based pump) or a variable speed pump (or a flow based or peristaltic pump) 112, for providing a vacuum/aspiration source, which, in the case of a variable speed pump 112, can be controlled by a pump speed controller 116. The unit 102 further includes an ultrasonic power source 114 and an ultrasonic power level controller 118 for controlling the power P applied to the needle 15 of the handpiece 104. A vacuum sensor 120 provides an input to the computer 110 representing the vacuum level on the output side of the pump 112. Venting may be provided by a vent 122.

The system 100 may also include a phase detector 124 for providing an input to the computer 100 that represents the phase between a sine wave representation of the voltage applied to the handpiece 104 and the resultant current into the handpiece 104. The functional representation of the system 100 also includes a system bus 126 to enable the various elements to be operably in communication with each other.

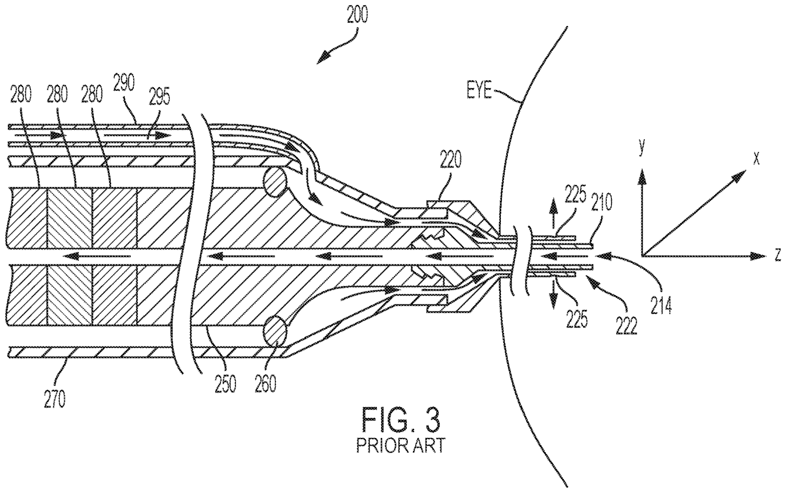

Turning to FIG. 3, the cross-section along the longitudinal axis of a portion of a phacoemulsification handpiece 200 known in the art is shown. Generally, the handpiece 200 includes a needle 210, defining a lumen that is operatively coupled to an aspiration pump (e.g. aspiration pump 40 (FIG. 1)), forming an aspiration line 214. At least a portion of the distal end of needle 210 is surrounded by sleeve 220 and proximal end of the needle 210 is coupled to a horn 250, which has its proximal end coupled to a set of piezoelectric crystals 280, shown as three rings. The horn 250, crystals 280, and a proximal portion of the needle 210 are enclosed within a handpiece casing 270 having an irrigation port coupled to an irrigation line 290 defining an irrigation pathway 295. Irrigation pathway 295 extends between the wall of sleeve 220 and the wall of needle 210, allowing fluid to flow around needle 210 and exit one or more ports 225 in sleeve 220. The irrigation line 290 is coupled to the irrigation source 30 (FIG. 1).

The horn 250 is typically an integrated metal, such as titanium, structure and often includes a rubber O-ring 260 around the mid-section, just before the horn 250 tapers to fit with the needle 210 at the horn's 250 distal end. The O-ring 260 snugly fits between the horn 250 and the casing 270. The O-ring 260 seals the proximal portion of the horn 250 from the irrigation pathway 295. Thus, there is a channel of air defined between the horn 250 and the casing 270. Descriptions of handpieces known in the art are provided in U.S. Pat. Nos. 6,852,092 (to Kadziauskas et al.) and 5,843,109 (to Mehta et al.).

In preparation for operation, a sleeve 220 is typically added to the distal end of the handpiece 200, covering the proximal portion of the needle 210 (thus, exposing the distal tip of the needle), and the distal end of the irrigation pathway 295, thereby extending the pathway 295 and defining an irrigation port 222 and/or port 225 just before the distal tip of the needle 210. The needle 210 and a portion of the sleeve 220 are then inserted through the cornea of the eye to reach the cataractic lens.

During operation, the irrigation path 295, the eye's chamber and the aspiration line 214 form a fluidic circuit, where irrigation fluid enters the eye's chamber via the irrigation path 295, and is then aspirated through the aspiration line 214 along with other materials that the surgeon desires to aspirate out, such as the cataractic lens. If, however, the materials, such as the cataractic lens, are too hard and massive to be aspirated through the aspiration line 214, then the distal end of the needle 210 is ultrasonically vibrated and applied to the material to be emulsified into a size and state that can be successfully aspirated.

The needle 210 is ultrasonically vibrated by applying electric power to the piezoelectric crystals 280, which in turn, cause the horn 250 to ultrasonically vibrate, which in turn, ultrasonically vibrates the needle 210. The electric power is defined by a number of parameters, such as signal frequency and amplitude, and if the power is applied in pulses, then the parameters can further include pulse width, shape, size, duty cycle, amplitude, and so on. These parameters are controlled by the control unit 102 and example control of these parameters is described in U.S. Pat. No. 7,169,123 to Kadziauskas et al.

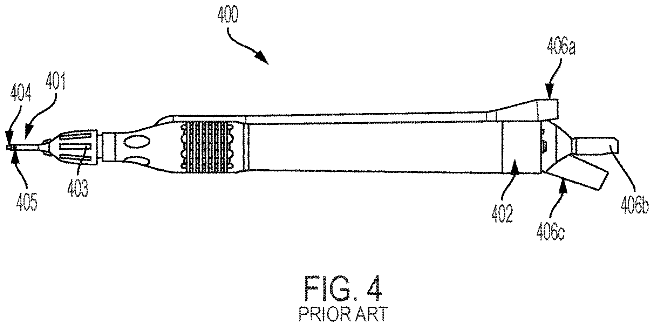

With respect to FIG. 4, an exemplary handpiece known in the prior art is shown. As discussed above, the distal end 401 of the handpiece 400 is show with a tip/needle 404 and sleeve 403 having port 405. The proximal end 402 of the of the handpiece 400 comprises multiple ports/connector points 406, include a port 406a for connecting to the irrigation line, a port 406b for connecting to the aspiration line, and a connector port 406c for electrical power for the ultrasound.

The location of the ports/connector points 406 at the proximal end 402 of the handpiece 400 are known to create fatigue on the surgeon's hand and wrist due to the invariability in the orientation of the ports/connector points 406 in light of the rigidly correspondent weight of the proximal end 402 once the irrigation and aspiration lines and the power cord are connected to the handpiece (not shown). This fatigue from orienting the distal end of the handpiece results, in part and as shown in FIG. 4, from the typical construction of the handpiece as one-piece metal-type material. Consequently, to adjust or rotate the distal end of the phacoemulsification (phaco) tip/needle requires the entire handpiece and connected lines to be moved/rotated in unison to achieve the desired position.

With regard to achieving the desired position, the emulsifying needle is often bent or has a bevel edge, and thus must be properly positioned to achieve emulsification of the lens. Further, the irrigation ports on the handpiece are optimally oriented so as to direct fluid along the horizontal plane of the eye. As such, in the known art, the surgeon will frequently rotate the handpiece such that the needle tip is at whatever angle is most proper to remove the cataract material, but unfortunately, due to the afore-discussed construction of the typical phacoemulsification handpiece, this rotation of the needle also executes a correspondent rotation away from the optimal position for the irrigation ports.

This need to move/rotate the entire handpiece also creates fatigue to the surgeon's hand and/or wrist during surgery. As such, a new handpiece with features that address these drawbacks is needed.
Therefore, the need exists for a phacoemulsification handpiece that allows for ergonomic rotational movement of the emulsifying needle separately from movement of the irrigation ports.

US 2008/0294087 A1 discloses a phacoemulsification system having a handpiece with a needle. US 2019/0321017 A1 discloses an ergonomic handpiece with two or more segments coupled together and capable of independently rotating. US 2010/0069825 A1 and US 2009/0005712 A1 disclose systems for controlling transverse phacoemulsification systems. US 2005/0277970 A1 discloses a surgical cutting instrument including an actuator assembly rotatably coupling a hub to a handpiece, where movement of the actuator is not co-axial with the hub axis. US 2016/0038342 A1 discloses a tympanostomy tube delivery device with a rotatable flexible shaft. WO 2011/008672 A2 discloses an electrosurgery generator for an ultrasonic surgical instrument.

### SUMMARY

The present invention provides a phacoemulsification handpiece as recited in claim 1. Optional features are recited in the dependent claims.

### BRIEF DESCRIPTION OF THE DRAWINGS

In order to better appreciate how the above-recited and other advantages and objects of the inventions are obtained, a more particular description of the instruments briefly described above will be rendered by reference to the accompanying drawings. It should be noted that the components in the figures are not necessarily to scale, emphasis instead being placed upon illustrating the principles of the disclosure. Moreover, in the figures, like reference numerals may or may not designate corresponding parts throughout the different views. Moreover, all illustrations are intended to convey concepts, where relative sizes, shapes and other detailed attributes may be illustrated schematically rather than literally or precisely. More specifically, in the drawings:
FIG. 1 is a diagram of a phacoemulsification system known in the art;
FIG. 2 is another diagram of a phacoemulsification system known in the art;
FIG. 3 is a diagram of a phacoemulsification handpiece known in the art;
FIG. 4 is an example of a phacoemulsification handpiece known in the art;
FIG. 5 shows a handpiece having a rotating tip;
FIG. 6 shows a handpiece having a rotating tip;
FIG. 7 shows a handpiece having a rotating tip;
FIG. 8 shows a handpiece having a rotating tip;
FIG. 9 shows an embodiment of a handpiece having a rotating tip; and
FIG. 10 shows an embodiment of a handpiece having a rotating tip.

### DETAILED DESCRIPTION

The figures and descriptions provided herein may be simplified to illustrate aspects of the described instruments, while eliminating for the purpose of clarity other aspects that may be found in typical surgical, and particularly ophthalmic surgical, devices, systems, and methods. Those of ordinary skill may thus recognize that other elements and/or steps may be desirable or necessary to implement the devices and systems described herein. Because such elements and steps are well known in the art, and because they do not facilitate a better understanding of the disclosed embodiments, a discussion of such elements and steps may not be provided herein. However, the present disclosure is deemed to inherently include all such elements, variations, and modifications to the described aspects that would be known to those of ordinary skill in the pertinent art.

Numerous specific details are set forth, such as examples of specific aspects and devices, to provide a thorough understanding of embodiments of the present disclosure. Nevertheless, it will be apparent to those skilled in the art that certain specific disclosed details need not be employed, and that embodiments may be embodied in different forms. As such, the exemplary embodiments set forth should not be construed to limit the scope of the disclosure.

The terminology used herein is for the purpose of describing particular embodiments only and is not intended to be limiting. For example, as used herein, the singular forms "a", "an" and "the" may be intended to include the plural forms as well, unless the context clearly indicates otherwise. The terms "comprises," "comprising," "including," and "having," are inclusive and therefore specify the presence of stated features, steps, operations, elements, and/or components, but do not preclude the presence or addition of one or more other features, steps, operations, elements, components, and/or groups thereof. The steps, processes, and operations described herein are not to be construed as necessarily requiring their respective performance in the particular order discussed or illustrated, unless specifically identified as a preferred or required order of performance. It is also to be understood that additional or alternative steps may be employed, in place of or in conjunction with the disclosed aspects.

When an element or layer is referred to as being "on", "upon", "connected to" or "coupled to" another element or layer, it may be directly on, upon, connected or coupled to the other element or layer, or intervening elements or layers may be present, unless clearly indicated otherwise. In contrast, when an element or layer is referred to as being "directly on," "directly upon", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). Further, as used herein the term "and/or" includes any and all combinations of one or more of the associated listed items.

Yet further, although the terms first, second, third, etc. may be used herein to describe various elements or aspects, these elements or aspects should not be limited by these terms. These terms may be only used to distinguish one element or aspect from another. Thus, terms such as "first," "second," and other numerical terms when used herein do not imply a sequence or order unless clearly indicated by the context. Thus, a first element, component, region, layer or section discussed below could be termed a second element, component, region, layer or section without departing from the teachings of the disclosure.

Certain types of ocular dysfunction, such as cataracts, are commonly treated with the surgical procedures referenced above, wherein the natural lens is removed from the eye and replaced with a clear artificial intraocular lens (IOL). More specifically, as the lens is emulsified under a vacuum pull, it is aspirated from the eye. Also, during the procedure, irrigation fluid is administered into the eye as the emulsified material is aspirated, thereby maintaining pressure in the interior of the eye.

The embodiments herein may relate to a standard, rigid ultrasonic handpiece, as detailed above. Additionally, the embodiments may relate to a handpiece having a multi-directional, such as flexurally moving, needle, or the embodiments may relate to a rotating handpiece.

More specifically, for each of the types of phacoemulsification handpieces referenced throughout-namely a standard, rigid handpiece, a rotationally-enabled handpiece, or a flexurally or multi-direction-capable handpiece-the embodiments provide ergonomic handpiece elements that allow for rotational movement of the emulsifying needle separately from movement of the irrigation sleeve, and/or of the needle and sleeve independently from the body of the handpiece. More particularly, the embodiments provide an external thumb wheel that controls rotation of at least the vibrating tip.

The embodiments may include an external transducer housing having a thumb wheel protruding therethrough. This actuatable thumb wheel may allow the surgeon to adjust the position of the phacoemulsification tip and/or sleeve, such as by rotating the position of the acoustic train that vibrates the needle tip. In short, the thumb wheel is attached or otherwise associated with the acoustic aspects of the tip at a flange thereof. Accordingly, the surgeon may achieve repeated and controlled rotation of the phacoemulsification tip with very minimal surgeon fatigue.

The thumb wheel may be attached to the transducer stack at the flange. However, the thumb wheel may also attach to an external casing that may seal the transducer for autoclaving. In such a circumstance, that external casing may then attach to the transducer stack at the flange.

Thus, the disclosed handpiece may be axially stationary, and/or may operate flexurally, and/or may rotate around its center axis, while the surgeon may independently make fine movements of the tip using the thumb wheel. Therefore, the limited movement requirements of the cables on the end of the handpiece necessary to achieve the desired rotational position of the phacoemulsification tip substantially reduce surgeon fatigue.

A rigid handpiece is detailed above. A rotationally enabled handpiece may have one or more rotatable segments in conjunction with managed, twistable cords and irrigation/aspiration lines, which allows for rotation of the phacoemulsification tip independent of these cords and lines.

In a flexurally-enabled handpiece, the ultrasonic horn may provide both longitudinal motion at the needle tip, and/or transversal/flexural motion at the needle tip, to emulsify the lens of the eye. The transversal motion provides a side-to-side or back-and-forth "sanding" motion at the tip to break up the lens and the longitudinal motion that causes any occluding particulate to move away from the tip.

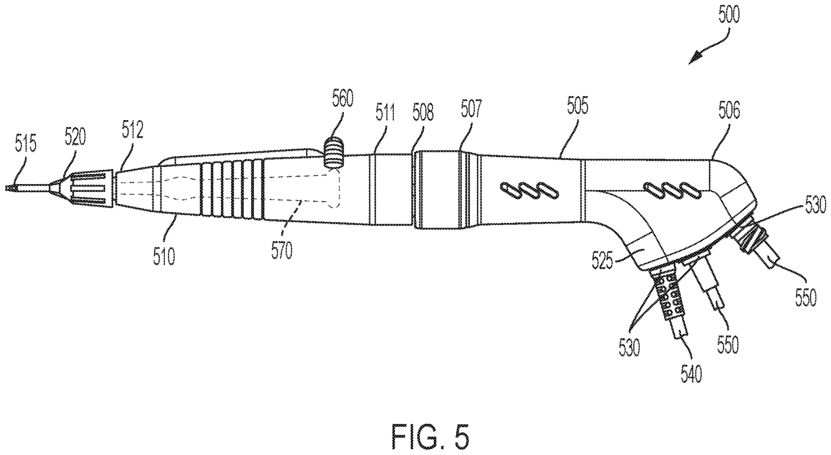

By way of example of a rotationally enabled handpiece, FIG. 5 illustrates that the handpiece 500 may have at least two segments, a proximal segment/portion 505 and a distal segment 510. Proximal segment 505 and distal segment 510 may be coupled to each other. Proximal segment 505 may have a first end 506 and a second end 507. Distal segment/portion 510 may have a first end 511 and a second end 512.

Proximal segment 505 may be coupled to distal segment 510 via the first end 511 and second end 507. Proximal segment 505 and second segment 510 may be coupled together by coupler 508 using any means known in the art, including, but not limited to a low friction stainless steel bearing that freely allows axial rotation between the proximal segment 505 and the distal segment 510, such as axial rotation up to 350 degrees. In an embodiment, the axial rotation may be up to 180 degrees. In another embodiment, the axial rotation may be up to 90 degrees.

The coupler 508 may reside between the first end 511 and the second end 507. In addition, the at least one coupler 508 may be a part of the proximal segment 505 or the distal segment 510, and provides a swivel feature that allows proximal segment 505 and distal segment 510 to rotate independently of one another about an axis A-A. In an embodiment, the proximal and/or distal segments may be capable of rotating up to 359 degrees.

In an embodiment, the distal segment 510 of handpiece 500 has a needle 515 connected to a distal-most portion of distal segment 510. A sleeve 520 may also be coupled with handpiece 500 and at least partially surround needle 515. Needle 515 and sleeve 520 may be separate components attachable to the distal segment 510 or may be integrally coupled with the distal segment 510 of handpiece 500. Proximal segment 505 of handpiece 500 includes tubing/cord management section 525 that includes one or more port/connector 530.

Needle 515, or needle 515 and irrigation sleeve 520, are coupled with a rotating element 560 on an upper aspect of the distal segment 510. Rotating element 560 is the thumb wheel 560 shown. The thumb wheel 560 is in communication with the transducer/horn 570 that vibrates needle 515 within the body of the distal segment 510, so as to rotate needle 515 upon actuation of thumb wheel 560 without rotation of other aspects of the handpiece 500. Alternatively, the thumb wheel 560 may also be in communication with the irrigation sleeve 520, so as to rotate both the irrigation sleeve 520 and needle 515 upon actuation of thumb wheel 560.

The one or more port/connector 530 has cords 540 and/or tubing 550 connected thereto. In the known art, these connected cords 540 and/or tubing 550 lays or rests against a user's hand or wrist as the distal segment 510 is moved about.

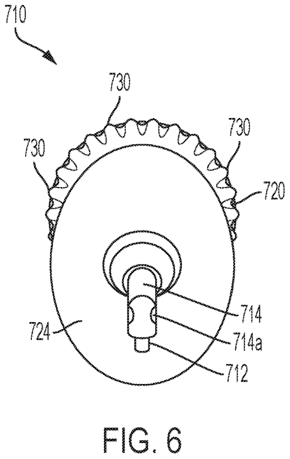

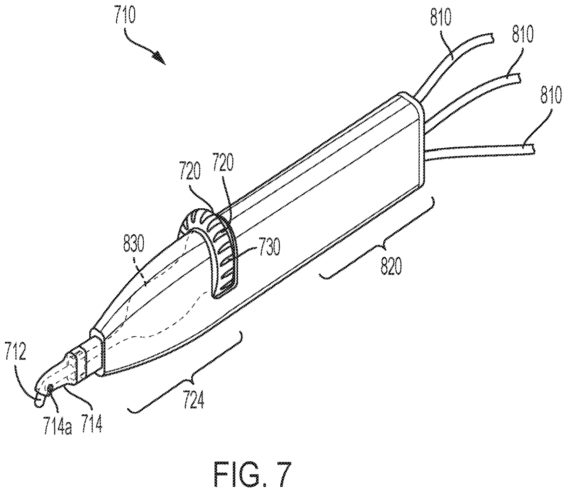

FIG. 6 illustrates a front view of a phacoemulsification handpiece 710, which may be the rigid handpiece discussed above or a rotationally-enabled handpiece 500, according to the embodiments. In the embodiment, the handpiece 710 shown includes an emulsifying tip 712 driven by an internal transducer stack and horn (not shown in FIG. 7), and irrigation sleeve 714 having one or more irrigation ports 714a.

Also shown is a thumb wheel 720 protruding from the uppermost portion (as shown) of the handpiece 710, proximate to the distal portion 724 of the handpiece 710. This thumb wheel 720 is associated in the body of the handpiece 710 with the transducer stack that drives the emulsifying tip 712.

Correspondingly, a rotation of the thumb wheel 720 rotates the horn to which the thumb wheel is connected. This rotation, in turn, rotates the emulsifying tip 712 driven by the transducer stack and horn. This rotation of the emulsifying tip 712 is therefore independent of the movement of the distal portion 724 of handpiece 710.

Also illustrated in FIG. 6 is a feedback aspect 730 on thumb wheel 720. The feedback aspect 730 may provide tactile feedback to the user as to the rotational position of the emulsifying tip 712. The feedback aspect 730 may thus take the form of texture, bumps or lines embedded on wheel 720. In alternative embodiments, feedback aspect 730 may take the form of an indicator window, in which indicator markings provide information to the surgeon as to the rotational position of the tip 712; or of a detent at the neutral position, and/or of varying detents indicative of the rotational position of tip 712.

FIG. 7 is an isometric view of the handpiece 710 having a thumb wheel 720. In the illustration, a plurality of cords/tubing 810, such as for feeding power, aspiration, and/or irrigation fluid are provided at the proximal portion 820 of the handpiece 710. It is these cords 810 that need not be rotated in order to rotate the tip 712 using thumb wheel 720 in the disclosed embodiments, thereby preventing surgeon fatigue.

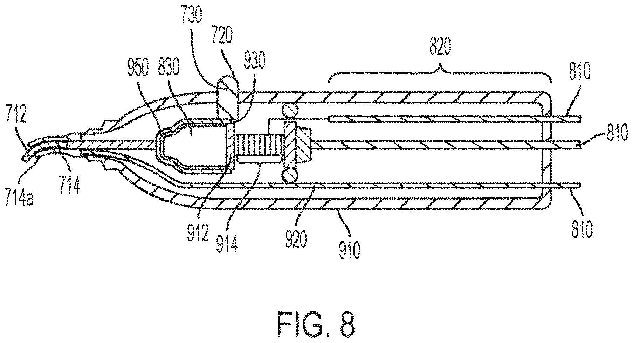

Also shown in FIG. 7 are the tip 712, the transducer stack/horn 830 that drives the tip 712, and the thumb wheel 720 having feedback aspect 730 and which is in rotational communication with the horn 830 (such as using the detent and/or the teeth referenced in the discussion of FIG. 8) opposite the tip 712 within the body of handpiece 710. One or more irrigation ports 714a of irrigation sleeve 714 at the distal portion 724 of the handpiece 710 are additionally shown.

FIG. 8 is a cross-sectional view of handpiece 710 not according to the invention. As illustrated, the thumb wheel 720 is connectively associated within the body 910 of handpiece 710 with the flange 912 coupled with horn 830 and/or transducer stack 914. Alternatively, transducer stack 914 and/or horn 830 may be encompassed by an optional casing 950 that includes a separate flange 912 coupled with the casing and capable of rotationally communicating with thumb wheel 720.

Of note, the thumb wheel 720 rotationally communicates with a flange 912, that resides on the horn 830, the stack 914, a casing encompassing the horn and/or the stack, and/or the irrigation sleeve 714 using a "notch" 930. Of course, the thumb wheel may be otherwise circumferentially incomplete to allow for the rotational association of the thumb wheel 720 with the flange 912 within the body housing 910. Yet further, the notch comprises a plurality of teeth which may, for example, "mate" with teeth on the flange 912 to allow the rotational force applied to the wheel 720 to be imparted to the flange 912.

Also of note in relation to the cross-section of FIG. 8, certain aspects within body 910 may be routed with the rotation of the flange 912 and wheel 720 in mind. For example, irrigation line 920 may be routed at or near the "bottom" area within the body 910 or at any other location in the handpiece, such that it does not travel around or adjacent to, or otherwise interfere with the operation of, the thumb wheel 720.

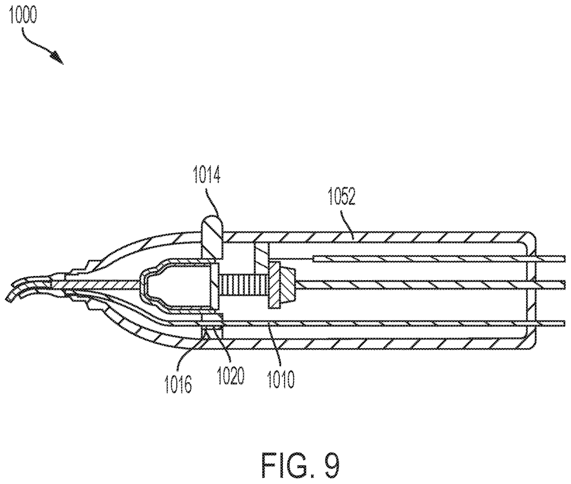

FIG. 9 is an additional cross-sectional illustration of a handpiece 1000 according to the invention. In the illustration, the irrigation line 1010 is routed through the thumb wheel 1014. The illustration shows one or more windows 1016 in the non-actuatable portion of the thumb wheel 1014, and it is through these one or more windows 1016 that the irrigation 1010 line, and/or any other line or connection passes. The skilled artisan will appreciate that this window 1016 may: be one or more in number; may be at a "lower" portion of the thumb wheel 1014 towards the center thereof; be placed and formed such that flexibility and/or rotatability of line 1010 is or is not required; and/or may include a low friction surface 1020 and/or padding about the open aspects thereof, so as not to cut through or otherwise damage line 1010 during repeated actuation of the thumb wheel 1014.

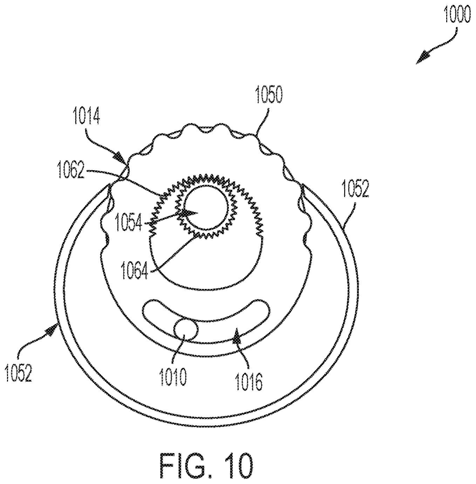

Similarly, FIG. 10 is a forward cross-sectional view of a handpiece 1000 according to the invention. In this illustration, the thumb wheel 1014 includes an actuatable upper portion 1050 extending outside of the housing 1052, which associates with the flange 1054 within the housing 1052 via one or more mating sets of gear teeth 1062, 1064. Also included in the illustration of FIG. 10 is a window 1016 through the "lower" portion of the thumb wheel 1014, i.e., on a portion thereof opposite the actuatable portion 1050 and thus "below" the portion of the thumb wheel 1014 through which the horn passes and in which the gear teeth 1062 of the thumb wheel associate with the gear teeth 1064 of the horn flange 1054. Through this window 1016 passes irrigation line 1010, and any other line or connections for phacoemulsification may also pass therethrough.

Although the disclosure has been described and illustrated in exemplary forms with a certain degree of particularity, it is noted that the description and illustrations have been made by way of example only. Numerous changes in the details of construction, combination, and arrangement of parts and steps may be made. Accordingly, such changes are intended to be included within the scope of the disclosure.

## Claims

1. A phacoemulsification handpiece (1000) comprising:
a proximal portion having a longitudinal axis, and a first end and a second end, wherein at least aspiration, irrigation and power inputs enter the first end;
a distal portion (1052) along the longitudinal axis and comprising, at a distalmost portion thereof from the proximal portion:
a needle configured to be vibrated by a transducer powered by the power input, the transducer residing within the distal portion and being associated with a horn; and **characterized in that**:
the horn has a circumferential flange (1054); and **in that** the handpiece further comprises:
a thumb wheel (1014) circumferentially associated with the circumferential flange (1054), and having a portion (1050) thereof extending out of the distal portion (1052) to allow for a user to rotationally actuate the extending portion (1050);
wherein rotational actuation of the thumb wheel (1014) affects a rotation of the circumferential flange (1054),
wherein the thumb wheel (1014) comprises:
a circumferential notch for association with the circumferential flange (1054) within the distal portion (1052) via one or more mating sets of gear teeth (1062, 1064), wherein the circumferential flange (1054) of the horn passes through the circumferential notch, and
a window (1016) through a portion of the thumb wheel (1014) that is opposite the extending portion (1050) and below the circumferential notch, wherein an irrigation line (1010) connected to the irrigation input passes through the window (1016).

2. The handpiece of claim 1, further comprising a feedback aspect (730) associated with the rotational actuation.

3. The handpiece of claim 2, wherein the feedback aspect (730) comprises tactile feedback.

4. The handpiece of claim 3, wherein the tactile feedback comprises one of embedded bumps or lines on the thumb wheel (1014).

5. The handpiece of claim 2, wherein the feedback aspect (730) comprises an indicator window through which markings are visible indicating rotational position.

6. The handpiece of claim 2, wherein the feedback aspect (730) comprises a wheel detent at a neutral position.

7. The handpiece of claim 2, wherein the feedback aspect (730) comprises varying wheel detents indicative of a plurality of rotational positions.

8. The handpiece of claim 1, wherein the notch comprises a plurality of teeth (1062) for mating with the flange (1054).

9. The handpiece of claim 8, wherein the flange (1054) comprises flange teeth (1064) correspondent to the teeth (1062).

10. The handpiece of claim 1, further comprising a rotating coupler (508) capable of coupling the proximal portion and the distal portion (1052) to enable independent axial rotation about the longitudinal axis of the proximal portion from the distal portion (1052).

11. The handpiece of claim 10, further comprising a plurality of flexible tubing (1010) passing substantially along the longitudinal axis within both the proximal portion and the distal portion (1052), wherein at least a first flexible tube of the plurality provides continuous fluidic communication between the aspiration input and an aspiration output of the distal portion, and the irrigation tube (1010) provides continuous fluidic communication between the irrigation input and an irrigation output in the distal portion, and wherein the plurality of flexible tubing flexes within the proximal portion and the distal portion (1052) and within the rotating coupler (508) so as not to bind during the independent axial rotation.

12. The handpiece of claim 1, wherein the transducer imparts multi-directional movement to the needle.

13. The handpiece of claim 1, wherein the proximal portion and the distal portion (1052) are rigidly associated along the longitudinal axis.

14. The handpiece of claim 11, wherein a fluidic connection between the aspiration input and an aspiration output in the distal portion is routed through the window in the thumb wheel.

15. The handpiece of claim 1, wherein the rotational actuation of the thumb wheel (1014) causes rotation of the horn and needle independent of movement of the aspiration, irrigation and power inputs.

## Patentansprüche

1. Phakoemulsifikationshandstück (1000), umfassend:
einen proximalen Abschnitt, der eine Längsachse und ein erstes und ein zweites Ende aufweist, wobei mindestens die Aspirations-, Spül- und Stromeingänge an dem ersten Ende eintreten;
einen distalen Abschnitt (1052) entlang der Längsachse, und, an einem distalsten Abschnitt davon von dem proximalen Abschnitt, umfassend:
eine Nadel, die konfiguriert ist, um durch einen Wandler in Vibration versetzt zu werden, der durch die Stromzufuhr mit Strom versorgt wird, wobei sich der Wandler innerhalb des distalen Abschnitts befindet und einem Horn zugeordnet ist; und **dadurch gekennzeichnet, dass:**
das Horn einen Umfangsflansch (1054) aufweist; und dadurch, dass das Handstück ferner umfasst:
ein Daumenrad (1014), das in Umfangsrichtung dem Umfangsflansch (1054) zugeordnet ist und einen Abschnitt (1050) davon aufweist, der sich aus dem distalen Abschnitt (1052) erstreckt, um einem Benutzer zu ermöglichen, den sich erstreckenden Abschnitt (1050) rotierend zu betätigen;
wobei eine Rotationsbetätigung des Daumenrads (1014) eine Rotation des Umfangsflansches (1054) bewirkt,
wobei das Daumenrad (1014) umfasst:
eine Umfangskerbe für eine Zuordnung mit dem Umfangsflansch (1054) innerhalb des distalen Abschnitts (1052) über einen oder mehrere zusammenpassende Sätze von Zahnrädern (1062, 1064), wobei der Umfangsflansch (1054) des Horns durch die Umfangskerbe passiert, und
ein Fenster (1016) durch einen Abschnitt des Daumenrads (1014), der dem sich erstreckenden Abschnitt (1050) gegenüberliegt und sich unterhalb der Umfangskerbe befindet, wobei eine Spülleitung (1010), die mit dem Spüleingang verbunden ist, durch das Fenster (1016) passiert.

2. Handstück nach Anspruch 1, ferner umfassend einen Rückmeldungsaspekt (730), der der Rotationsbetätigung zugeordnet ist.

3. Handstück nach Anspruch 2, wobei der Rückmeldungsaspekt (730) eine taktile Rückmeldung umfasst.

4. Handstück nach Anspruch 3, wobei die taktile Rückmeldung eines von eingebetteten Erhebungen oder Linien auf dem Daumenrad (1014) umfasst.

5. Handstück nach Anspruch 2, wobei der Rückmeldungsaspekt (730) ein Anzeigefenster umfasst, durch das Markierungen sichtbar sind, die eine Rotationsposition anzeigen.

6. Handstück nach Anspruch 2, wobei der Rückmeldungsaspekt (730) eine Radarretierung in einer neutralen Position umfasst.

7. Handstück nach Anspruch 2, wobei der Rückmeldungsaspekt (730) unterschiedliche Radarretierungen umfasst, die eine Vielzahl von Rotationspositionen anzeigen.

8. Handstück nach Anspruch 1, wobei die Kerbe eine Vielzahl von Zähnen (1062) zum Zusammenpassen mit dem Flansch (1054) umfasst.

9. Handstück nach Anspruch 8, wobei der Flansch (1054) Flanschzähne (1064) aufweist, die den Zähnen (1062) entsprechen.

10. Handstück nach Anspruch 1, ferner umfassend einen Rotationskoppler (508), der in der Lage ist, den proximalen Abschnitt und den distalen Abschnitt (1052) zu koppeln, um eine unabhängige axiale Rotation des proximalen Abschnitts von dem distalen Abschnitt (1052) um die Längsachse herum zu aktivieren.

11. Handstück nach Anspruch 10, ferner umfassend eine Vielzahl von flexiblen Schläuchen (1010), die im Wesentlichen entlang der Längsachse sowohl innerhalb des proximalen Abschnitts als auch des distalen Abschnitts (1052) passieren, wobei mindestens ein erster flexibler Schlauch der Vielzahl eine kontinuierliche fluidische Kommunikation zwischen dem Aspirationseingang und einem Aspirationsausgang des distalen Abschnitts bereitstellt, und der Spülschlauch (1010) eine kontinuierliche fluidische Kommunikation zwischen dem Spüleingang und einem Spülausgang in dem distalen Abschnitt bereitstellt, und wobei sich die Vielzahl von flexiblen Schläuchen innerhalb des proximalen Abschnitts und des distalen Abschnitts (1052) und innerhalb des Rotationskopplers (508) biegt, um während der unabhängigen axialen Rotation nicht zu verklemmen.

12. Handstück nach Anspruch 1, wobei der Wandler der Nadel eine multidirektionale Bewegung verleiht.

13. Handstück nach Anspruch 1, wobei der proximale Abschnitt und der distale Abschnitt (1052) entlang der Längsachse starr zugeordnet sind.

14. Handstück nach Anspruch 11, wobei eine fluidische Verbindung zwischen dem Aspirationseingang und einem Aspirationsausgang in dem distalen Abschnitt durch das Fenster in dem Daumenrad geführt wird.

15. Handstück nach Anspruch 1, wobei die Rotationsbetätigung des Daumenrads (1014) eine Rotation des Horns und der Nadel unabhängig von der Bewegung der Aspirations-, Spül- und Stromeingänge veranlasst.

## Revendications

1. Pièce à main de phacoémulsification (1000) comprenant :
une partie proximale ayant un axe longitudinal, et une première extrémité et une seconde extrémité, dans laquelle au moins des entrées d'aspiration, d'irrigation et de puissance entrent dans la première extrémité ;
une partie distale (1052) le long de l'axe longitudinal et comprenant, au niveau de sa partie la plus distale par rapport à la partie proximale :
une aiguille conçue pour être mise en vibration par un transducteur alimenté par l'entrée de puissance, le transducteur résidant dans la partie distale et étant associé à un pavillon ; et **caractérisé en ce que** :
le pavillon a une collerette circonférentielle (1054) ; et **en ce que** la pièce à main comprend en outre :
une molette (1014) associée de manière circonférentielle à la collerette circonférentielle (1054), et ayant une partie (1050) de celle-ci s'étendant hors de la partie distale (1052) pour permettre à un utilisateur d'actionner par rotation la partie s'étendant (1050) ;
dans laquelle l'actionnement rotatif de la molette (1014) affecte une rotation de la collerette circonférentielle (1054),
dans laquelle la molette (1014) comprend :
une encoche circonférentielle pour une association avec la collerette circonférentielle (1054) à l'intérieur de la partie distale (1052) par l'intermédiaire d'un ou plusieurs ensembles de dents d'engrenage accouplés (1062, 1064), dans laquelle la collerette circonférentielle (1054) du pavillon passe à travers l'encoche circonférentielle, et
une fenêtre (1016) à travers une partie de la molette (1014) qui est opposée à la partie d'extension (1050) et en dessous de l'encoche circonférentielle, dans laquelle une ligne d'irrigation (1010) reliée à l'entrée d'irrigation passe à travers la fenêtre (1016).

2. Pièce à main selon la revendication 1, comprenant en outre un aspect de rétroaction (730) associé à l'actionnement rotatif.

3. Pièce à main selon la revendication 2, dans laquelle l'aspect de rétroaction (730) comprend une rétroaction tactile.

4. Pièce à main selon la revendication 3, dans laquelle la rétroaction tactile comprend l'une parmi des bosses ou des lignes encastrées sur la molette (1014).

5. Pièce à main selon la revendication 2, dans laquelle l'aspect de rétroaction (730) comprend une fenêtre d'indicateur à travers laquelle des marques sont visibles pour indiquer une position de rotation.

6. Pièce à main selon la revendication 2, dans laquelle l'aspect de rétroaction (730) comprend un cran de roue en position neutre.

7. Pièce à main selon la revendication 2, dans laquelle l'aspect de rétroaction (730) comprend des crans de roue variables indiquant une pluralité de positions de rotation.

8. Pièce à main selon la revendication 1, dans laquelle l'encoche comprend une pluralité de dents (1062) pour s'accoupler avec la collerette (1054).

9. Pièce à main selon la revendication 8, dans laquelle la collerette (1054) comprend des dents de collerette (1064) correspondant aux dents (1062).

10. Pièce à main selon la revendication 1, comprenant en outre un coupleur rotatif (508) capable d'accoupler la partie proximale et la partie distale (1052) pour permettre une rotation axiale indépendante autour de l'axe longitudinal de la partie proximale par rapport à la partie distale (1052).

11. Pièce à main selon la revendication 10, comprenant en outre une pluralité de tubes souples (1010) passant sensiblement le long de l'axe longitudinal à la fois dans la partie proximale et la partie distale (1052), dans laquelle au moins un premier tube souple de la pluralité assure une communication fluidique continue entre l'entrée d'aspiration et une sortie d'aspiration de la partie distale, et le tube d'irrigation (1010) assure une communication fluidique continue entre l'entrée d'irrigation et une sortie d'irrigation dans la partie distale, et dans laquelle la pluralité de tubes souples fléchit à l'intérieur de la partie proximale et de la partie distale (1052) et à l'intérieur du coupleur rotatif (508) de manière à ne pas se lier pendant la rotation axiale indépendante.

12. Pièce à main selon la revendication 1, dans laquelle le transducteur transmet un mouvement multidirectionnel à l'aiguille.

13. Pièce à main selon la revendication 1, dans laquelle la partie proximale et la partie distale (1052) sont associées de manière rigide le long de l'axe longitudinal.

14. Pièce à main selon la revendication 11, dans laquelle une liaison fluidique entre l'entrée d'aspiration et une sortie d'aspiration dans la partie distale est acheminée à travers la fenêtre de la molette.

15. Pièce à main selon la revendication 1, dans laquelle l'actionnement rotatif de la molette (1014) entraîne la rotation du pavillon et de l'aiguille indépendamment du mouvement des entrées d'aspiration, d'irrigation et de puissance.
